# EUROPEAN PATENT SPECIFICATION

(11) **EP 3 813 769 B1**
(45) Date of publication and mention of the grant of the patent: **07.10.2026**
(21) Application number: 19733081.4
(22) Date of filing: 28.06.2019
(51) Int. Cl.: A61K 8/19, A61K 8/92, A61K 8/37, A61K 8/31, A61K 8/58, A61Q 15/00, A61K 8/04

(54) **DEODORANT COMPOSITION IN AEROSOL FORM**
DESODORIERENDE ZUSAMMENSETZUNG IN AEROSOLFORM
COMPOSITION DÉODORANTE SOUS FORME D'AÉROSOL

(30) Priority: 29.06.2018 FR 1856025
(43) Date of publication of application: 05.05.2021
(73) Proprietor: L'OREAL, 75008 Paris (FR)
(72) Inventor: MOUJAHED, Zohra, 94152 CHEVILLY LA RUE (FR); BOUARFA, Bouchra, 94550 Chevilly-Larue (FR)
(74) Representative: Ipsilon
(86) International application number: PCT/EP2019/067456
(87) International publication number: WO 2020/002665

(56) References cited:
- WO-A2-2012/085055
- DE-A1- 102008 012 457
- DE-A1- 102008 035 014
- DE-A1- 102009 027 050
- DE-A1- 102011 083 872
- FR-A1- 2 992 553
- FR-A1- 3 053 591
- US-A- 4 659 560
- US-A1- 2010 143 273
- US-A1- 2016 296 428
- US-A1- 2017 007 510
- DATABASE GNPD [online] MINTEL; 23 September 2005 (2005-09-23), ANONYMOUS: "Toiletries Kit", XP055585850, retrieved from www.gnpd.com Database accession no. 10232728

## Description

The present invention relates to the field of compositions, notably cosmetic compositions, preferably deodorant compositions, as defined in the set of claims.

More particularly, the invention relates to the field of caring for and cleansing the skin, in particular bodily skin.

The present invention also relates to a process for the cosmetic treatment of the skin for treating the body odour associated with human perspiration, notably underarm odour, and optionally human perspiration.

In the cosmetics field, it is well known to use, in topical application, deodorant products containing active substances of antiperspirant type or of deodorant type for reducing or even preventing body odour, notably underarm odour, which is generally unpleasant.

Eccrine or apocrine sweat generally has little odour when it is secreted. It is its degradation by bacteria *via* enzymatic reactions which produces malodorous compounds. Deodorant active agents thus have the function of reducing or preventing the formation of unpleasant odours. This aim can be achieved notably by means of deodorant and/or antiperspirant activity.

In this respect, various systems may be proposed, such as unpleasant-odour absorbers, bactericidal substances, substances for blocking the enzymatic reactions responsible for the formation of the odorous compounds or neutralization of the volatile compounds responsible for the odour.

For example, aluminium and/or zirconium salts are used as antibacterial agents. These salts play a direct role on the deodorant efficacy by reducing the number of bacteria responsible for the degradation of sweat.

Deodorant products are generally available in the form of roll-ons, tubes, sticks, aerosols or sprays. Aerosols, which are often favoured by consumers, are fatty presentation forms that leave a greasy feel under the armpit, and some of which have a tendency to transfer onto clothing, leaving visible and unaesthetic marks.

In order notably to overcome the problem of marks, deodorant compositions containing oils with a refractive index close to those of aluminium salts have been developed. The role of such oils is to reduce the whitish appearance of antiperspirant compositions when they are deposited on the skin and, consequently, to make the marks on clothing less white.

However, such deodorant compositions have the drawback of giving the skin, notably on the armpits, an oily feel that is unpleasant for the user, and do not make it possible to limit the transfer of deodorant products from the skin onto clothing.

Anhydrous deodorant compositions containing magnesium salts in combination with specific oils have also been proposed. These compositions advantageously make it possible to reduce the white marks. They are not entirely satisfactory in sensory terms, however.

Other compositions have also been proposed, notably in DE 10 2009 027050, WO 2012/085055, FR 3 053 591, DE 10 2008 035014, US 2016/296428, US 4,659,560, DE 10 2008 012457, US 2010/143273, FR 2 992 553, US 2017/007510, and DE 10 2011 083872. However, the described compositions are not satisfactory.

Moreover, nowadays, consumers are constantly in search of products whose effects on the environment and on health are reduced. In this respect, formulations comprising the least possible amount of ingredients are increasingly favoured.

However, all of the deodorant products available in aerosol form contain numerous additional compounds, such as silicone oils or modified clays, in order to obtain compositions that are satisfactory in sensory terms.

Thus, the omission of such additional compounds gives rise to drawbacks on application and produces a deposit that is less dry and more greasy.

Consequently, there is an ongoing need for novel methods for treating the body odour associated with human perspiration, notably underarm odour, which do not have the set of drawbacks described above.

In particular, there is a need for deodorant compositions in aerosol form which leave a dry and non-greasy feel under the armpit, while at the same time being free of additional compounds, and in particular of silicone oils.

The aim of the present invention is to satisfy these needs.

Thus, according to a first aspect, the present invention is directed towards an anhydrous aerosol composition, having a water content of less than 5% by weight relative to the total weight of the composition, notably a cosmetic composition, comprising:
- at least 0.3% by weight of active material of at least one deodorant active agent, relative to the total weight of the composition, the at least one deodorant active agent(s) comprising magnesium salts;
- at least two hydrocarbon-based oils, the at least two hydrocarbon-based oils comprising at least one hydrocarbon-based plant oil, and at least one fatty acid ester different from hydrocarbon-based plant oils; and
- at least one propellant;
characterized in that the composition comprises less than 0.5% by weight of silicone oil(s), and less than 0.1% by weight of modified clay(s), relative to the total weight of the composition, and in that the mass ratio of hydrocarbon-based oil(s) relative to the total amount of oils present in the composition is between 0.85 and 1.00.

Contrary to all expectation, the inventors have in fact discovered that the combination of these compounds makes it possible to limit the formation of malodorous underarm odours, while at the same time advantageously reducing the tack. They thus make it possible to obtain a dry and non-greasy deposit.

Advantageously, these effects are afforded in the absence of additional compounds, such as silicone oils, and/or modified clays.

Furthermore, the present invention provides compositions which transfer less onto textiles, thereby generating fewer visible and unaesthetic marks on clothing, notably on dark-coloured clothing, relative to the compositions already known.

Thus, the compositions, notably the cosmetic compositions, according to the invention have good transfer-resistance and also deodorant properties.

According to a preferred embodiment, the present invention relates to an anhydrous aerosol composition, notably a cosmetic composition, comprising:
- at least 0.3% by weight of active material of deodorant active agent(s) relative to the total weight of the composition, the at least one deodorant active agent(s) comprising magnesium salts;
- at least coconut oil;
- at least one fatty acid ester; and
- at least one propellant;
characterized in that the composition comprises less than 0.5% by weight of silicone oil(s), relative to the total weight of the composition, and in that the mass ratio of fatty acid ester(s) relative to the total amount of oils present in the composition is between 0.55 and 0.85.

According to another preferred embodiment, the present invention relates to an anhydrous aerosol composition, notably a cosmetic composition, comprising:
- at least 0.3% by weight of active material of deodorant active agent(s) relative to the total weight of the composition, the at least one deodorant active agent(s) comprising magnesium salts;
- at least one hydrocarbon-based plant oil;
- at least isopropyl palmitate; and
- at least one propellant;
characterized in that the composition comprises less than 0.5% by weight of silicone oil(s), relative to the total weight of the composition, and in that the mass ratio of fatty acid ester(s) relative to the total amount of oils present in the composition is between 0.55 and 0.85.

According to yet another preferred embodiment, the present invention relates to an anhydrous aerosol composition, notably a cosmetic composition, comprising:
- at least 0.3% by weight of active material of magnesium salt(s) relative to the total weight of the composition;
- at least coconut oil;
- at least isopropyl palmitate; and
- at least one propellant;
characterized in that the composition comprises less than 0.5% by weight of silicone oil(s), relative to the total weight of the composition, and in that the mass ratio of fatty acid ester(s) relative to the total amount of oils present in the composition is between 0.55 and 0.85.

According to yet another preferred embodiment, the present invention relates to an anhydrous aerosol composition, notably a cosmetic composition, comprising:
- at least 0.3% by weight of active material of magnesium oxide relative to the total weight of the composition;
- at least coconut oil;
- at least isopropyl palmitate; and
- at least one propellant;
characterized in that the composition comprises less than 0.5% by weight of silicone oil(s), relative to the total weight of the composition, and in that the mass ratio of fatty acid ester(s) relative to the total amount of oils present in the composition is between 0.55 and 0.85. The composition of the invention as claimed comprises less than 0.1% by weight of modified clay(s), relative to the total weight of the composition.

According to another preferred embodiment, the present invention relates to an anhydrous aerosol composition, notably a cosmetic composition, comprising:
- at least 0.3% by weight of active material of magnesium salt(s) relative to the total weight of the composition;
- at least two hydrocarbon-based oils; and
- at least one propellant;
characterized in that the composition comprises less than 0.5% by weight of silicone oil(s), relative to the total weight of the composition, and in that the mass ratio of hydrocarbon-based oil(s) relative to the total amount of oils present in the composition is between 0.85 and 1.00.

The present disclosure, not encompassed by the wording of the claims, relates to an anhydrous aerosol composition, notably a cosmetic composition, comprising:
- at least 0.3% by weight of active material of magnesium salt(s) relative to the total weight of the composition;
- at least one hydrocarbon-based oil; and
- at least one propellant;
characterized in that the composition comprises less than 0.5% by weight of silicone oil(s), relative to the total weight of the composition, comprises less than 0.1% by weight of modified clay(s) relative to the total weight of the composition, comprises less than 0.1% by weight of hydrophobic fumed silica(s) relative to the total weight of the composition, and in that the mass ratio of hydrocarbon-based oil(s) relative to the total amount of oils present in the composition is between 0.85 and 1.00.

The present disclosure, not encompassed by the wording of the claims, relates to an anhydrous aerosol composition, notably a cosmetic composition, comprising:
- at least 0.3% by weight of active material of magnesium salt(s) relative to the total weight of the composition;
- at least coconut oil; and
- at least one propellant;
characterized in that the composition comprises less than 0.5% by weight of silicone oil(s), relative to the total weight of the composition, and in that the mass ratio of hydrocarbon-based oil(s) relative to the total amount of oils present in the composition is between 0.85 and 1.00.

The present disclosure, not encompassed by the wording of the claims, relates to an anhydrous aerosol composition, notably a cosmetic composition, comprising:
- at least 0.3% by weight of active material of magnesium salt(s) relative to the total weight of the composition;
- at least two hydrocarbon-based oils; and
- at least one propellant;
characterized in that the composition comprises less than 0.5% by weight of silicone oil(s), relative to the total weight of the composition, comprises less than 0.1% by weight of modified clay(s) relative to the total weight of the composition, comprises less than 0.1% by weight of hydrophobic fumed silica(s) relative to the total weight of the composition, and in that the mass ratio of hydrocarbon-based oil(s) relative to the total amount of oils present in the composition is between 0.85 and 1.00.

According to another preferred embodiment, the present invention relates to an anhydrous aerosol composition, notably a cosmetic composition, comprising:
- at least 0.3% by weight of active material of magnesium salt(s) relative to the total weight of the composition;
- at least coconut oil,
- at least one fatty acid ester;
- at least one propellant;
characterized in that the composition comprises less than 0.5% by weight of silicone oil(s), relative to the total weight of the composition, and in that the mass ratio of hydrocarbon-based oil(s) relative to the total amount of oils present in the composition is between 0.85 and 1.00.

The composition of the invention as claimed comprises less than 0.1% by weight of modified clay(s), relative to the total weight of the composition.

The present disclosure, not encompassed by the wording of the claims, relates to an anhydrous aerosol composition, notably a cosmetic composition, comprising:
- at least 0.3% by weight of active material of magnesium salt(s) relative to the total weight of the composition;
- at least coconut oil,
- at least one propellant;
characterized in that the composition comprises less than 0.5% by weight of silicone oil(s), relative to the total weight of the composition, comprises less than 0.1% by weight of modified clay(s) relative to the total weight of the composition, comprises less than 0.1% by weight of hydrophobic fumed silica(s) relative to the total weight of the composition, and in that the mass ratio of hydrocarbon-based oil(s) relative to the total amount of oils present in the composition is between 0.85 and 1.00.

According to another preferred embodiment, the present invention relates to an anhydrous aerosol composition, notably a cosmetic composition, comprising:
- at least 0.3% by weight of active material of magnesium salt(s) relative to the total weight of the composition;
- at least coconut oil,
- at least one fatty acid ester;
- at least one propellant;
characterized in that the composition comprises less than 0.5% by weight of silicone oil(s), relative to the total weight of the composition, comprises less than 0.1% by weight of modified clay(s) relative to the total weight of the composition, comprises less than 0.1% by weight of hydrophobic fumed silica(s) relative to the total weight of the composition, and in that the mass ratio of hydrocarbon-based oil(s) relative to the total amount of oils present in the composition is between 0.85 and 1.00.

Moreover, a subject of the present invention is also, according to another of its aspects, a process for the cosmetic treatment of body odour associated with human perspiration, notably underarm odour, and optionally human perspiration, comprising the step of applying a composition as defined above to a skin surface.

The process of the invention is particularly advantageous for treating body odour associated with perspiration under the armpits, since the composition used does not give an unpleasant greasy feel and transfers less onto clothing.

The compositions, notably the cosmetic compositions, according to the invention comprise a physiologically acceptable medium.

For the purposes of the present invention, the term "physiologically acceptable medium" is intended to denote a medium that is suitable for the administration of a composition to the skin.

A physiologically acceptable medium generally has no unpleasant odour or appearance, and is entirely compatible with topical administration to the skin. In the present case, where the composition is intended for application to the surface of the skin, such a medium is considered in particular to be physiologically acceptable when it does not cause any stinging, tautness or redness that is unacceptable to the user.

In particular, the composition is suitable for application to the surface of the skin. Thus, the physiologically acceptable medium is preferentially a cosmetically or dermatologically acceptable medium, i.e. a medium that has no unpleasant odour, colour or appearance, and that does not cause the user any unacceptable stinging, tautness or redness.

The composition may then comprise any constituent usually used in the envisaged application.

Needless to say, a person skilled in the art will take care to select this or these optional additional compound(s), and/or the amount thereof, such that the advantageous properties of the compounds according to the invention are not, or are not substantially, adversely affected by the envisaged addition.

For the purposes of the present invention, the term "anhydrous" refers to a composition having a water content of less than 5% by weight, in particular less than 2% by weight, preferably less than 1% by weight, or even less than 0.5% by weight, relative to the total weight of the composition. Even more preferentially, a composition according to the invention is totally free of water.

It should be noted that the water that may be present is more particularly bound water, such as water of crystallization in salts, or traces of water absorbed by the starting materials used in the preparation of the compositions of the invention.

Other characteristics, aspects and advantages of the invention will become apparent on reading the detailed description which follows.

### DEODORANT ACTIVE AGENTS

As indicated previously, a composition according to the invention comprises at least 0.3% by weight of active material of at least one deodorant active agent relative to the total weight of the composition.

The term "deodorant active agent" means any substance that is capable of reducing, masking or absorbing human body odour, in particular underarm odour.

Preferably, the deodorant active agent(s) are present in a content ranging from 0.3% to 10% by weight and more preferably in a content ranging from 0.5% to 8% by weight of active material relative to the total weight of the composition.

The contents are expressed relative to the total composition, which includes the gas comprised in the case of an aerosol.

The deodorant active agent(s) are chosen from magnesium salts.

In particular, a composition according to the invention comprises at least 0.3% by weight of active material of at least one magnesium salt relative to the total weight of the composition.

The term "magnesium salts" comprises magnesium derivatives including magnesium oxides.

The magnesium salt is chosen in particular from magnesium oxide, magnesium carbonate, magnesium hydroxide, magnesium bicarbonate, magnesium chloride, magnesium sulfate, magnesium acetate, magnesium pidolate, magnesium gluconate, magnesium glutamate, magnesium heptagluconate, magnesium ketogluconate, magnesium lactate, magnesium ascorbate, magnesium citrate, magnesium aspartate, magnesium pantothenate, magnesium sorbate, magnesium nitrate, magnesium lactate gluconate, magnesium fulvate, and mixtures thereof.

Use will be made more particularly of magnesium oxide, magnesium carbonate, magnesium hydroxide or magnesium bicarbonate and mixtures thereof, and even more preferentially magnesium oxide.

According to one embodiment, the magnesium salts are present in a content ranging from 0.3% to 5% by weight, preferably in a content ranging from 0.5% to 4% by weight of active material relative to the total weight of the composition.

In particular, the proportion of magnesium salts relative to the total weight of the composition (excluding gas) preferably ranges from 2% to 30%, preferably from 3% to 20% by weight.

In particular, the proportion of magnesium salts relative to the total amount of oils, in a composition according to the invention, ranges in particular from 5% to 30% by weight and preferably from 10% to 25% by weight.

Deodorant active agents that may also be mentioned include bacteriostatic agents or bactericidal agents acting on the microorganisms of underarm odours, for instance 2,4,4'-trichloro-2'-hydroxydiphenyl ether (^{®}Triclosan), 2,4-dichloro-2'-hydroxydiphenyl ether, 3',4',5'-trichlorosalicylanilide, 1-(3',4'-dichlorophenyl)-3-(4'-chlorophenyl)urea (Triclocarban^{®}) or 3,7,11-trimethyldodeca-2,5,10-trienol (Farnesol^{®}); quaternary ammonium salts such as cetyltrimethylammonium salts, cetylpyridinium salts, DPTA (1,3-diaminopropanetetraacetic acid), 1,2-decanediol (Symclariol from the company Symrise); glycerol derivatives, for instance caprylic/capric glycerides (Capmul MCM^{®} from Abitec), glyceryl caprylate or caprate (Dermosoft GMCY^{®} and Dermosoft GMC^{®} from Straetmans), polyglyceryl-2 caprate (Dermosoft DGMC^{®} from Straetmans), and biguanide derivatives, for instance polyhexamethylene biguanide salts; chlorhexidine and salts thereof; 4-phenyl-4,4-dimethyl-2-butanol (Symdeo MPP^{®} from Symrise); zinc salts such as zinc salicylate, zinc gluconate, zinc pidolate, zinc sulfate, zinc chloride, zinc lactate or zinc phenolsulfonate; salicylic acid and derivatives thereof such as 5-n-octanoylsalicylic acid.

Other deodorant active agents that may be mentioned are odour absorbers such as zinc ricinoleates, sodium bicarbonate; metallic or silver or silver-free zeolites, or cyclodextrins and derivatives thereof.

Other deodorant active agents that may be mentioned are chelating agents such as Dissolvine GL-47-S^{®} from AkzoNobel, EDTA and DPTA. It may also be a polyol such as glycerol or 1,3-propanediol (Zemea Propanediol sold by Dupont Tate and Lyle BioProducts); or an enzyme inhibitor such as triethyl citrate; or alum.

Other deodorant active agents that may also be mentioned are bacteriostatic agents or bactericides, 2,4,4'-trichloro-2'-hydroxydiphenyl ether (Triclosan^{®}), 2,4-dichloro-2'-hydroxydiphenyl ether, 3',4',5'-trichlorosalicylanilide, 1-(3',4'-dichlorophenyl)-3-(4'-chlorophenyl)urea (Triclocarban^{®}) or 3,7,11-trimethyldodeca-2,5,10-trienol (Farnesol^{®}); quaternary ammonium salts such as cetyltrimethylammonium salts or cetylpyridinium salts.

### HYDROCARBON-BASED OIL

The composition according to the invention comprises at least two hydrocarbon-based oils, the at least two hydrocarbon-based oils comprising at least one hydrocarbon-based plant oil, and at least one fatty acid ester different from hydrocarbon-based plant oils.

In particular, a composition according to the invention comprises from 0.5% to 30% by weight of hydrocarbon-based oil(s), preferably from 1% to 20% by weight of hydrocarbon-based oil(s) and even more preferentially from 1% to 18% by weight of hydrocarbon-based oil(s) relative to the total weight of the composition.

Preferably, the mass ratio of hydrocarbon-based oil(s) relative to the total amount of oils present in the composition is between 0.90 and 1.00 and is preferably equal to 1.

Hydrocarbon-based oils that may notably be mentioned include:
- hydrocarbon-based oils of plant origin;
- linear or branched hydrocarbons, of mineral or synthetic origin, such as liquid paraffins and derivatives thereof, petroleum jelly, polydecenes, polybutenes, hydrogenated polyisobutene such as Parleam, or squalane;
- synthetic ethers containing from 10 to 40 carbon atoms, such as dicaprylyl ether;
- polyol esters and pentaerythritol esters, for instance dipentaerythrityl tetrahydroxystearate/tetraisostearate;
- esters of diol dimers and of diacid dimers, such as Lusplan DD-DA5^{®} and Lusplan DD-DA7^{®} sold by the company Nippon Fine Chemical and described in patent application US 2004-175 338;
- copolymers of diol dimer and of diacid dimer and esters thereof, such as dilinoleyl diol dimer/dilinoleic dimer copolymers, and esters thereof;
- copolymers of polyols and of diacid dimers, and esters thereof, such as Hailuscent ISDA or the dilinoleic acid/butanediol copolymer;
- fatty alcohols that are liquid at room temperature, with a branched and/or unsaturated carbon-based chain containing from 12 to 26 carbon atoms, for instance 2-octyldodecanol, isostearyl alcohol, oleyl alcohol, 2-hexyldecanol, 2-butyloctanol and 2-undecylpentadecanol;
- C₁₂-C₂₂ higher fatty acids, such as oleic acid, linoleic acid, linolenic acid, and mixtures thereof;
- dialkyl carbonates, the two alkyl chains possibly being identical or different, such as dicaprylyl carbonate sold under the name Cetiol CC^{®} by Cognis;
- fatty acid esters; and
- mixtures thereof.

Preferably, the hydrocarbon-based plant oils are natural, and optionally biological.

Besides the properties provided to the composition according to the invention, such an oil makes it possible to reduce the white marks that may be left after the application of a deodorant and/or antiperspirant composition.

The hydrocarbon-based oil is chosen in particular from liquid triglycerides of fatty acids containing 4 to 24 carbon atoms, for instance heptanoic or octanoic acid triglycerides, or wheatgerm oil, olive oil, sweet almond oil, palm oil, rapeseed oil, coconut oil, cottonseed oil, alfalfa oil, poppy oil, pumpkin oil, marrow oil, blackcurrant oil, evening primrose oil, millet oil, barley oil, quinoa oil, rye oil, safflower oil, candlenut oil, passionflower oil, musk rose oil, sunflower oil, corn oil, soybean oil, grapeseed oil, sesame seed oil, hazelnut oil, apricot oil, macadamia oil, castor oil, avocado oil, caprylic/capric acid triglycerides, for instance those sold by the company Stéarineries Dubois or those sold under the names Miglyol 810, 812 and 818 by the company Dynamit Nobel, jojoba oil and shea butter oil.

Hydrocarbon-based plant oils that may in particular be mentioned include:
- coconut oil, derived notably from *Cocos nucifera,* having a mass per unit volume of between 0.907 and 0.909 g.cm⁻³, a saponification number between 255 and 267, and an iodine number between 5 and 9;
- babassu oil (from kernel), derived notably from palm trees, having a mass per unit volume of between 0.914 and 0.917 g.cm⁻³, a saponification number between 245 and 256, and an iodine number between 10 and 18;
- palm kernel oil, derived notably from *Elaeis guineensis,* having a mass per unit volume of between 0.903 and 0.908 g.cm⁻³, a saponification number between 246 and 254, and an iodine number between 12 and 19;
- cocoa butter, derived notably from *Theobroma cacao,* having a mass per unit volume of between 0.906 and 0.909 g.cm⁻³, a saponification number between 192 and 200, and an iodine number between 33 and 40;
- palm oil (from pulp), derived notably from *Elaeis guineensis,* having a mass per unit volume of between 0.897 and 0.900 g.cm⁻³, a saponification number between 195 and 205, and an iodine number between 45 and 48;
- baobab kernel oil, derived notably from *Adansonia grandideris,* having a mass per unit volume of between 0.895 and 0.905 g.cm⁻³, a saponification number between 190 and 195, and an iodine number between 57 and 63;
- shea butter derived notably from *Sapotacea multiflora,* having a mass per unit volume of between 0.900 and 0.902 g.cm⁻³, a saponification number between 178 and 193, and an iodine number between 52 and 66;
- illipé butter, derived notably from *Skorea stenoptera,* having a mass per unit volume of between 0.902 and 0.905 g.cm⁻³, a saponification number between 190 and 194, and an iodine number between 58 and 65;
- olive oil (from pulp and stone), derived notably from *Olea europea,* having a mass per unit volume of between 0.910 and 0.916 g.cm⁻³, a saponification number between 182 and 196, and an iodine number between 75 and 94;
- peanut oil, derived notably from *Arachis hypogea,* having a mass per unit volume of between 0.914 and 0.917 g.cm⁻³, a saponification number between 187 and 196, and an iodine number between 80 and 106;
- almond oil, derived notably from *Prunus amygdalis,* having a mass per unit volume of between 0.911 and 0.917 g.cm⁻³, a saponification number between 189 and 196, and an iodine number between 95 and 103;
- hazelnut oil, derived notably from *Corylu avelana,* having a mass per unit volume of between 0.914 and 0.920 g.cm⁻³, a saponification number between 190 and 195, and an iodine number between 83 and 110;
- rapeseed oil, derived notably from *Brassica napus,* having a mass per unit volume of between 0.910 and 0.920 g.cm⁻³, a saponification number between 168 and 181, and an iodine number between 94 and 120; and
- rice bran oil, derived notably from *Oriza sativa,* having a mass per unit volume of between 0.920 and 0.924 g.cm⁻³, a saponification number between 180 and 194, and an iodine number between 85 and 109.

In the claimed invention, one of the hydrocarbon-based oil is a hydrocarbon-based plant oil, in particular chosen from castor oil, sweet almond oil and coconut oil, and mixtures thereof.

Preferably, the hydrocarbon-based plant oil is coconut oil.

Coconut oil is a mixture of various acids, and notably of caproic acid, caprylic acid, capric acid, lauric acid, myristic acid, palmitic acid, stearic acid, oleic acid, linoleic acid and trans fatty acids.

In the composition according to the invention, one of the hydrocarbon-based oil(s) are chosen from fatty acid esters. Fatty acid esters are different from hydrocarbon-based plant oils.

In other terms, in a composition according to the invention, the hydrocarbon-based oil(s) are chosen from fatty acid esters different from hydrocarbon-based plant oils.

Fatty acid esters that may notably be mentioned include synthetic fatty acid esters, for instance the oils of formula R₁COOR₂ in which R₁ represents the residue of a linear or branched higher fatty acid comprising from 1 to 40 carbon atoms and R₂ represents a hydrocarbon-based chain, which is notably branched, containing from 1 to 40 carbon atoms, with R₁ + R₂ ≥ 10, for instance purcellin oil (cetostearyl octanoate), isononyl isononanoate, isopropyl myristate, isopropyl palmitate, C₁₂ to C₁₅ alkyl benzoate, hexyl laurate, diisopropyl adipate, 2-ethylhexyl palmitate, 2-octyldodecyl stearate, 2-octyldodecyl erucate, isostearyl isostearate or tridecyl trimellitate; alcohol or polyalcohol octanoates, decanoates or ricinoleates, for instance propylene glycol dioctanoate; hydroxylated esters, for instance isostearyl lactate, octyl hydroxystearate, octyldodecyl hydroxystearate, diisostearyl malate, triisocetyl citrate, and fatty alcohol heptanoates, octanoates or decanoates; polyol esters, for instance propylene glycol dioctanoate, neopentyl glycol diheptanoate or diethylene glycol diisononanoate; and pentaerythritol esters, for instance pentaerythrityl tetraisostearate.

Preferably, the fatty acid ester is chosen from isopropyl palmitate, isopropyl myristate, isononyl isononanoate and C₁₂-C₁₅ alkyl benzoate, and mixtures thereof.

Fatty acid esters such as isopropyl palmitate and isopropyl myristate, and mixtures thereof, and even more particularly isopropyl palmitate, will be chosen more preferentially.

A composition according to the invention comprises at least two hydrocarbon-based oils, namely at least one hydrocarbon-based plant oil, in particular coconut oil, and at least one fatty acid ester.

In other terms, according to a preferred embodiment, a composition according to the invention comprises at least two hydrocarbon-based oils, namely at least one hydrocarbon-based plant oil, in particular coconut oil, and at least one fatty acid ester different from hydrocarbon-based plant oils.

Advantageously, a composition according to the invention preferably comprises from 0.5% to 20% by weight of hydrocarbon-based plant oil(s), preferably from 1% to 15% by weight of hydrocarbon-based plant oil(s) and even more preferentially from 1% to 10% by weight of hydrocarbon-based plant oil(s) relative to the total weight of the composition.

Preferably, a composition according to the invention, comprises from 0.5% to 20% by weight of fatty acid ester(s), preferably from 1% to 18% by weight of fatty acid ester(s) and better still from 2% to 15% by weight of fatty acid ester(s) relative to the total weight of the composition.

Preferably, the composition according to the invention comprises at least one fatty acid ester and the mass ratio of fatty acid ester(s) relative to the total amount of oils present in the composition is between 0.55 and 0.85, in particular between 0.55 and 0.75 and more preferentially between 0.60 and 0.70.

Hydrocarbon-based oils that may also be mentioned include volatile hydrocarbon-based oils.

According to one embodiment, a composition according to the invention comprises at least one volatile hydrocarbon-based oil.

For the purposes of the invention, the term "volatile oil" means an oil that is capable of evaporating on contact with the skin or the keratin fibre in less than one hour, at room temperature and atmospheric pressure.

The volatile hydrocarbon-based oils of the invention are volatile cosmetic oils, which are liquid at room temperature, having a non-zero vapour pressure, at room temperature and atmospheric pressure, ranging in particular from 0.13 Pa to 40 000 Pa (10⁻³ to 300 mmHg), in particular ranging from 1.3 Pa to 13 000 Pa (0.01 to 100 mmHg) and more particularly ranging from 1.3 Pa to 1300 Pa (0.01 to 10 mmHg).

Examples of volatile hydrocarbon-based oils that may be mentioned include volatile hydrocarbon-based oils chosen from hydrocarbon-based oils containing from 8 to 16 carbon atoms, and notably C₈-C₁₆ isoalkanes of petroleum origin (also known as isoparaffins), for instance isododecane (also known as 2,2,4,4,6-pentamethylheptane), isodecane and isohexadecane, for example the oils sold under the trade names Isopar or Permethyl, branched C₈-C₁₆ esters and isohexyl neopentanoate, and mixtures thereof.

Other volatile hydrocarbon-based oils, for instance petroleum distillates, notably those sold under the name Shell Solt by the company Shell, may also be used, and also volatile linear alkanes, such as those described in patent application DE 10 2008 012 457 from the company Cognis.

According to a particular embodiment, a composition according to the invention comprises less than 1% by weight of volatile hydrocarbon-based oil(s), preferably less than 0.5% by weight of volatile hydrocarbon-based oil(s), relative to the total weight of the composition, and more preferentially is free of volatile hydrocarbon-based oil(s).

### OILY PHASE

The composition according to the invention comprises at least one oily phase containing at least two hydrocarbon-based oil, the at least two hydrocarbon-based oils comprising at least one hydrocarbon-based plant oil, and at least one fatty acid ester different from hydrocarbon-based plant oils. The composition according to the invention comprises less than 0.5% by weight of silicone oil(s) relative to the total weight of the composition.

The term "oil" means a fatty substance that is liquid at room temperature (25°C) and atmospheric pressure (760 mmHg, i.e. 10⁵ Pa).

As indicated previously, the composition according to the invention comprises less than 0.5% by weight of silicone oil(s), relative to the total weight of the composition; preferably, it comprises less than 0.2% by weight of silicone oil(s) relative to the total weight of the composition, and more preferentially is free of silicone oil(s).

Examples of volatile silicone oils that may be mentioned include:
- volatile linear or cyclic silicone oils, notably those with a viscosity ≤ 8 centistokes (8×10⁻⁶ m²/s) and notably containing from 2 to 7 silicon atoms, these silicones optionally comprising alkyl or alkoxy groups containing from 1 to 10 carbon atoms. As volatile silicone oils that may be used in the invention, mention may be made notably of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane;
- the volatile linear alkyltrisiloxane oils of general formula (I): 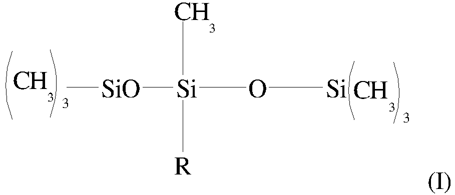 in which R represents an alkyl group containing from 2 to 4 carbon atoms and of which one or more hydrogen atoms may be substituted with one or more fluorine or chlorine atoms.

Among the oils of general formula (I), mention may be made of:
- 3-butyl-1,1,1,3,5,5,5-heptamethyltrisiloxane,
- 3-propyl-1,1,1,3,5,5,5-heptamethyltrisiloxane, and
- 3-ethyl-1,1,1,3,5,5,5-heptamethyltrisiloxane,
corresponding to the oils of formula (I) for which R is, respectively, a butyl group, a propyl group or an ethyl group.

As examples of non-volatile silicone oils, mention may be made of silicone oils such as linear or cyclic nonvolatile polydimethylsiloxanes (PDMSs), polydimethylsiloxanes comprising alkyl, alkoxy or phenyl groups, which are on the side or at the end of a silicone chain, these groups notably containing from 2 to 24 carbon atoms, and mixtures thereof.

The composition according to the invention may comprise at least one non-volatile oil, different from the hydrocarbon-based oil(s) already present in the composition.

The term "non-volatile oil" means an oil that remains on the skin or the keratin fibre at room temperature and atmospheric pressure for at least several hours, and that notably has a vapour pressure strictly less than 10⁻³ mmHg (0.13 Pa).

The non-volatile oil may be chosen from non-volatile hydrocarbon-based oils, non-volatile silicone oils and non-volatile fluoro oils, and mixtures thereof.

As examples of non-volatile fluoro oils, mention may be made of optionally partially hydrocarbon-based and/or silicone-based fluoro oils, such as fluorosilicone oils, fluorinated polyethers and fluorosilicones as described in EP 847 752.

### ADDITIVES

The cosmetic compositions according to the invention may also comprise cosmetic adjuvants chosen from moisture absorbers, lipophilic suspension agents or gelling agents, softeners, antioxidants, opacifiers, stabilizers, neutralizers, moisturizers, emollients or collagen-protecting agents, vitamins, UV stabilizers, bactericides, antiseptic agents, preserving agents, polymers, fragrances, essential oils, thickeners or suspension agents or any other ingredient usually used in cosmetics for this type of application.

The composition according to the invention may also comprise lipophilic thickeners, gelling agents and/or suspension agents to improve the texture or the homogeneity of the products.

It is a matter of routine operation for those skilled in the art to adjust the nature and the amount of the additives present in the compositions in accordance with the invention.

Needless to say, a person skilled in the art will take care to select this or these optional additional compounds such that the advantageous properties intrinsically associated with the composition in accordance with the invention are not, or are not substantially, adversely affected by the envisaged addition(s).

### MOISTURE ABSORBERS

It is also possible to add moisture absorbers, for instance perlites and preferably expanded perlites.

The cosmetic composition may comprise one or more moisture absorbers chosen from perlites.

Preferably, the cosmetic composition comprises one or more absorbers chosen from expanded perlites.

The perlites that may be used according to the invention are generally aluminosilicates of volcanic origin and have the composition:
- 70.0-75.0% by weight of silica SiO₂;
- 12.0-15.0% by weight of aluminium oxide Al₂O₃;
- 3.0-5.0% of sodium oxide Na₂O;
- 3.0-5.0% of potassium oxide K₂O;
- 0.5-2% of iron oxide Fe₂O₃;
- 0.2-0.7% of magnesium oxide MgO;
- 0.5-1.5% of calcium oxide CaO; and
- 0.05-0.15% of titanium oxide TiO₂.

The perlite is ground, dried and then calibrated in a first step. The product obtained, known as perlite ore, is grey-coloured and has a size of about 100 µm.

The perlite ore is then expanded (1000°C/2 seconds) to give more or less white particles. When the temperature reaches 850-900°C, the water trapped in the structure of the material evaporates and brings about the expansion of the material, relative to its original volume. The expanded perlite particles in accordance with the invention may be obtained via the expansion process described in patent US 5 002 698.

Preferably, the perlite particles used will be ground; in this case, they are known as Expanded Milled Perlite (EMP). They preferably have a particle size defined by a median diameter D50 ranging from 0.5 to 50 µm and preferably from 0.5 to 40 µm. Preferably, the perlite particles used have an untamped apparent density at 25°C ranging from 10 to 400 kg/m³ (standard DIN 53468) and preferably from 10 to 300 kg/m³.

Preferably, the expanded perlite particles according to the invention have a water absorption capacity, measured at the wet point, ranging from 200% to 1500% and preferably from 250% to 800%.

The wet point corresponds to the amount of water which has to be added to 1 g of particle in order to obtain a homogeneous paste. This method is directly derived from the oil uptake method applied to solvents. The measurements are taken in the same manner by means of the wet point and the flow point, which have, respectively, the following definitions:
wet point: weight expressed in grams per 100 g of product corresponding to the production of a homogeneous paste during the addition of a solvent to a powder;
flow point: weight expressed in grams per 100 g of product at and above which the amount of solvent is greater than the capacity of the powder to retain it. This is reflected by the production of a more or less homogeneous mixture which flows over the glass plate.

The wet point and the flow point are measured according to the following protocol:

*Protocol for measuring the water absorption*
*1) Equipment used*
   Glass plate (25 x 25 mm)
   Spatula (wooden shaft and metal part (15 x 2.7 mm))
   Silk-bristled brush
   Balance
*2) Procedure*

The glass plate is placed on the balance and 1 g of perlite particles is weighed out. The beaker containing the solvent and the liquid sampling pipette is placed on the balance. The solvent is gradually added to the powder, the whole being regularly blended (every 3 to 4 drops) by means of the spatula.

The mass of solvent needed to obtain the wet point is noted. Further solvent is added and the mass which makes it possible to reach the flow point is noted. The average of three tests will be determined.

The expanded perlite particles sold under the trade names Optimat 1430 OR or Optimat 2550 by the company World Minerals will be used in particular.

### FILLERS

The compositions in accordance with the invention may also comprise at least one filler of organic or mineral nature.

The term "filler" should be understood as meaning colourless or white solid particles of any form, which are in an insoluble form dispersed in the medium of the composition. These particles, of mineral or organic nature, give body or rigidity to the composition and/or softness and uniformity to the makeup.

The fillers used in the compositions according to the present invention may be in lamellar, globular or spherical form, in the form of fibres or in any other intermediate form between these defined forms.

The fillers according to the invention may or may not be surface-coated, and in particular they may be surface-treated with silicones, amino acids, fluorinated derivatives or any other substance which promotes the dispersion and the compatibility of the filler in the composition.

Examples of mineral fillers that may be mentioned include talc, mica, silica, hollow silica microspheres, kaolin, calcium carbonate, magnesium carbonate, hydroxyapatite, boron nitride, glass or ceramic microcapsules, or composites of silica and of titanium dioxide.

Examples of organic fillers that may be mentioned include polyamide powders (Nylon^{®} Orgasol from Atochem), polyethylene powders, polymethyl methacrylate powders, polytetrafluoroethylene (Teflon) powders, acrylic acid copolymer powders (Polytrap from the company Dow Corning), lauroyl lysine, hollow polymer microspheres such as those of polyvinylidene chloride/acrylonitrile, for instance Expancel (Nobel Industrie), hexamethylene diisocyanate/trimethylol hexyllactone copolymer powder (Plastic Powder from Toshiki), silicone resin microbeads (for example Tospearls from Toshiba), synthetic or natural micronized waxes, metal soaps derived from organic carboxylic acids containing from 8 to 22 carbon atoms and preferably from 12 to 18 carbon atoms, for example zinc stearate, magnesium stearate, lithium stearate, zinc laurate or magnesium myristate, Polypore^{®} L 200 (Chemdal Corporation), powders of crosslinked elastomeric organopolysiloxane coated with silicone resin, notably with silsesquioxane resin, as described, for example, in patent US 5 538 793, polyurethane powders, in particular powders of crosslinked polyurethane comprising a copolymer, said copolymer comprising trimethylol hexyllactone. It may in particular be a hexamethylene diisocyanate/trimethylol hexyllactone polymer. Such particles are notably commercially available, for example, under the name Plastic Powder D-400^{®} or Plastic Powder D-800^{®} from the company Toshiki, and mixtures thereof.

Preferably, the compositions according to the invention comprise at least one silicone filler, and in particular the product sold under the name KSG16^{®} by the company Shin-Etsu.

### MINERAL THICKENERS, GELLING AGENTS AND SUSPENSION AGENTS

As lipophilic mineral thickener, gelling agent or suspension agent, use may be made of modified clays that are preferably chosen from hydrophobic-modified montmorillonite clays, for instance hydrophobic-modified bentonites or hectorites. Examples that may be mentioned include the product Stearalkonium Bentonite (CTFA name) (product of reaction of bentonite and the quaternary ammonium stearalkonium chloride) such as the commercial product sold under the name Tixogel MP 250 by the company Sud Chemie Rheologicals, United Catalysts Inc. or the product Disteardimonium Hectorite (CTFA name) (product of reaction of hectorite and distearyldimonium chloride) sold under the name Bentone 38 or Bentone Gel by the company Elementis Specialities.

The composition according to the invention comprises less than 0.1% by weight of modified clay(s) relative to the total weight of the composition, and more preferentially is free of modified clay(s).

Mention may also be made of fumed silica optionally subjected to a hydrophobic surface treatment, the particle size of which is less than 1 µm. Indeed, it is possible to chemically modify the surface of the silica, by chemical reaction generating a reduction in the number of silanol groups present at the surface of the silica. It is notably possible to substitute silanol groups with hydrophobic groups: a hydrophobic silica is then obtained. The hydrophobic groups may be trimethylsiloxyl groups, which are obtained notably by treating fumed silica in the presence of hexamethyldisilazane. Silicas thus treated are known as "silica silylate" according to the CTFA (8th Edition, 2000). They are sold, for example, under the references Aerosil R812^{®} by the company Degussa, Cab-O-Sil TS-530^{®} by the company Cabot, dimethylsilyloxyl or polydimethylsiloxane groups, which are obtained notably by treating fumed silica in the presence of polydimethylsiloxane or dimethyldichlorosilane. Silicas thus treated are known as "Silica Dimethyl Silylate" according to the CTFA (8th edition, 2000). They are sold, for example, under the references Aerosil R972^{®} and Aerosil R974^{®} by the company Degussa and Cab-O-Sil TS-610^{®} and Cab-O-Sil TS-720^{®} by the company Cabot.

The hydrophobic fumed silica in particular has a particle size that may be nanometric to micrometric, for example ranging from about 5 to 200 nm.

Preferably, a composition according to the invention comprises less than 0.1% by weight of hydrophobic fumed silica(s) relative to the total weight of the composition, and more preferentially is free of modified clay(s).

### ANTIPERSPIRANT ACTIVE AGENT

According to a particular embodiment, the composition according to the invention may also comprise at least one antiperspirant active agent.

The term "antiperspirant active agent" means a salt which, by itself, has the effect of reducing the flow of sweat, of reducing the sensation on the skin of moisture associated with human sweat or of masking human sweat.

The cosmetic composition may in particular comprise one or more antiperspirant active agents chosen from aluminium and/or zirconium salts or complexes.

Among the aluminium salts or complexes, mention may be made of aluminium halohydrates.

Among the aluminium salts, mention may in particular be made of aluminium chlorohydrate, aluminium chlorohydrex, the aluminium chlorohydrex-polyethylene glycol complex, the aluminium chlorohydrex-propylene glycol complex, aluminium dichlorohydrate, the aluminium dichlorohydrex-polyethylene glycol complex, the aluminium dichlorohydrex-propylene glycol complex, aluminium sesquichlorohydrate, the aluminium sesquichlorohydrex-polyethylene glycol complex, the aluminium sesquichlorohydrex-propylene glycol complex, aluminium sulfate buffered with sodium aluminium lactate.

Aluminium sesquichlorohydrate is notably sold under the trade name Reach 301^{®} by the company SummitReheis.

Aluminium chlorohydrate is notably sold under the trade names Locron S FLA^{®}, Locron P and Locron L.ZA by the company Clariant; under the trade names Microdry Aluminum Chlorohydrate^{®}, Micro-Dry 323^{®}, Chlorhydrol 50, Reach 103 and Reach 501 by the company SummitReheis; under the trade name Westchlor 200^{®} by the company Westwood; under the trade name Aloxicoll PF 40^{®} by the company Guilini Chemie; Cluron 50%^{®} by the company Industria Quimica Del Centro; or Clorohidroxido Aluminio SO A 50%^{®} by the company Finquimica.

Aluminium chlorohydrate, aluminium sesquichlorohydrate and mixtures thereof will more particularly be used.

The aluminium salts or complexes may be present in the composition according to the invention in a content ranging from 0.2% to 50% by weight, preferably in a content ranging from 1% to 20% by weight and more particularly between 2% and 15% by weight relative to the total weight of the composition as oils.

Preferably, a composition according to the invention comprises less than 2% by weight of aluminium salts or complexes, more preferentially less than 1% by weight of aluminium salts or complexes and better still less than 0.5% by weight of aluminium salts or complexes relative to the total weight of the composition.

Preferably, a composition according to the invention is free of aluminium salts or complexes.

### PROPELLANT

According to the invention, the composition comprises one or more propellants.

The propellant used in the composition according to the invention is chosen from dimethyl ether, volatile hydrocarbons such as propane, isopropane, n-butane, isobutane, n-pentane and isopentane, and mixtures thereof, optionally with at least one chloro and/or fluoro hydrocarbon. Among the latter, mention may be made of the compounds sold by the company DuPont de Nemours under the names Freon^{®} and Dymel^{®}, and in particular monofluorotrichloromethane, difluorodichloromethane, tetrafluorodichloroethane and 1,1-difluoroethane, sold in particular under the trade name Dymel 152 A^{®} by the company DuPont.

Use may also be made, as propellant, of carbon dioxide gas, nitrous oxide, nitrogen or compressed air.

Preferably, the composition according to the invention comprises a propellant chosen from volatile hydrocarbons.

More preferentially, the propellant is chosen from isopropane, n-butane, isobutane, n-pentane and isopentane, and mixtures thereof, and is preferably isobutane.

In particular, the weight ratio between the liquid phase and the propellant gas varies in a ratio from 5/95 to 50/50, preferably from 10/90 to 40/60 and more preferentially from 15/85 to 30/70.

Preferably, the composition according to the invention comprises from 5% to 95% by weight of propellant(s), preferably from 20% to 95% by weight of propellant(s) and better still from 40% to 95% by weight of propellant(s) relative to the total weight of the composition.

### PRESENTATION FORMS

The anhydrous compositions according to the invention are aerosol compositions.

Thus, the compositions of the invention may be packaged in pressurized form in an aerosol device. In this regard, they contain the ingredients generally used in products of this type, which are well known to those skilled in the art.

The compositions according to the invention may be prepared by a person skilled in the art, according to the conventionally known methods.

The compositions may be in liquid, gel, semi-solid, solid or loose or compact powder form.

The invention also relates to a cosmetic process for treating body odour associated with human perspiration, notably underarm odour, and optionally human perspiration, which consists in applying to the surface of the skin an effective amount of the cosmetic composition as described previously.

The application time of the cosmetic composition to the surface of the skin may range from 0.5 to 10 seconds and preferably from 1 to 5 seconds.

The composition in accordance with the invention may be applied several times to the surface of the skin.

In particular, the cosmetic treatment process according to the invention consists in applying to the surface of the armpits an effective amount of the cosmetic composition as described above.

The disclosure, not encompassed by the wording of the claims, also relates to the use of said composition for the cosmetic treatment of body odour associated with human perspiration, notably underarm odour, and optionally human perspiration.

It also described an aerosol device consisting of a container comprising an aerosol composition as defined above and of a means for dispensing said composition.

The dispensing means, which forms a part of the aerosol device, generally consists of a dispensing valve controlled by a dispensing head, which itself comprises a nozzle via which the aerosol composition is vaporized. The container containing the pressurized composition may be opaque or transparent. It may be made of glass, polymer or metal, optionally coated with a protective varnish coat.

Throughout the description, including the claims, the term "including a" should be understood as being synonymous with "including at least one", unless otherwise specified.

The expressions "between ... and ...", and "ranging from ... to ..." should be understood as meaning limits included, unless otherwise specified.

In the description and the examples, the percentages are percentages by weight, unless otherwise indicated. The percentages are thus expressed by weight with respect to the total weight of the composition. The temperature is expressed in degrees Celsius, unless otherwise indicated, and the pressure is atmospheric pressure, unless otherwise indicated.

It is also understood that, unless otherwise mentioned, the contents are expressed relative to the weight of the final composition according to the invention, i.e. taking into account the presence of propellant gas.

The invention is illustrated in greater detail by the nonlimiting examples presented below.

### EXAMPLES

Three deodorant compositions in aerosol form according to the invention and outside the invention were prepared according to the procedure described below.

The composition of the formulations is detailed in table 1 below.

The amounts indicated are weight percentages relative to the total weight of the composition.

**Table 1**

| **Ingredients** | **Trade name** | **INCI name** | **Composition A** | **Composition B** |
|---|---|---|---|---|
| Distearyldimethylammonium-modified hectorite | Bentone 38VCG from Elementis | Disteardimonium hectorite | 1.67 | / |
| Magnesium oxide | Magnesium Oxide Light from Dr Paul Lohmann | Magnesium oxide | 10.00 | 15.00 |
| Refined coconut kernel oil | Refined coconut kernel oil GV 24/26 from Sio (ADM) | *Cocos nucifera* oil | 13.33 | 25.00 |
| Isopropyl palmitate | Isopropyl palmitate from Cognis (BASF) | Isopropyl palmitate | 33.13 | 49.00 |
| Fragrance | / | Fragrance | 7.33 | 11.00 |
| Polydimethylsiloxane (viscosity: 10 cSt) | Xiameter PMX-200 Silicone Fluid 10 CS from Dow Corning | Dimethicone | 10.00 | / |
| α,ω-Dihydroxylated polydimethylsiloxane/polydim ethylsiloxane mixture 5 cSt; | Xiameter PMX-1503 Fluid from Dow Corning | Dimethicone (and) Dimethiconol | 4.00 | / |
| Propylene carbonate | Jeffsol propylene from Huntsman | Propylene carbonate | 0.54 | / |
| Isododecane | Isododecane from Ineos | Isododecane | 20.00 | / |

The compositions are prepared by preliminary mixing of the oils with stirring.

The magnesium oxide is then dispersed in the mixture at high shear. The other ingredients are then added at high shear.

Finally, the aerosols are prepared by pressurization by mixing the compositions with the liquefied gas, in the amounts indicated in Table 2 below.

**Table 2**

| **Ingredients** | **Composition 1 (according to the invention)** | **Composition 2 (according to the invention)** | **Composition 3 (outside the invention)** |
|---|---|---|---|
| Composition A | / | / | 15.00 |
| Composition B | 10.00 | 20.00 | / |
| Propellant: Isobutane | 90.00 | 80.00 | 85.00 |

Compositions 1 and 3 are intended to be packaged in a "standard" 200 mL aerosol can. Composition 2 is intended to be packaged in a "compressed" 100 mL aerosol can.

### Results:

### Method for evaluating the "dry effect"

An aluminium pan 102 mm in diameter (*102 mm aluminium low-sided weighing pan - reference 11754059 Fisher Scientific)* is placed vertically on a support.

After vigorous shaking, the test aerosol is positioned vertically 20 cm from this pan. The formula is sprayed for about 8 seconds, making a circular motion of about 10 cm to obtain a uniform deposit on the surface of the pan of between 300 and 600 mg.

The pan is immediately weighed on a precision balance (*Mettler Toledo balance AX204 SNR1121393680)* and is then placed horizontally on a hotplate adjusted to 35°C (*SP Equation Hotplate - 800*400 model).* The weight loss is quantified by weighing the pan every 20 seconds for 3 minutes and then every minute for 12 minutes. The weight of the pan is measured.

The results are expressed as percentages of weight loss (relative to the initial weight) according to the formula: % loss = ((initial weight - measured weight)/initial weight) × 100

Six tests are performed for each formula.

### Method for evaluating the "greasy effect"

The test composition is sprayed onto a sample of Supplale^{®} *(Support Supplale - reference PBZ13001 from Idemitsu)* 24 mm in diameter, for 5 seconds, so as to be able to have a uniform deposit of product.

After having placed a stencil with an aperture of diameter 24 on the sample of Supplale^{®} positioned vertically, the aerosol to be evaluated, shaken vigorously beforehand, is positioned vertically 20 cm from this target. The formula is sprayed for 5 seconds, in a lateral motion with an amplitude of 4 cm, to obtain a uniform deposit on the Supplale^{®}. The fume cupboard suction is switched off so as not to influence the trajectory of the spray.

The sample thus coated with a film of the composition to be evaluated is then positioned on a hotplate set at 35°C. After 5 minutes, the sample is placed on the support of the table (x,α) of an automated spreader which is coupled to a texturometer onto which is attached a Roll'On Mouss roller. A piece of KimTech sheet *(KIMTECH Science Sheet - Precision wipes 11x21 - Kimberly-Clark^{®} Professionnal*) of dimensions 5 cm × 5 cm is positioned on the sample and the roller applies the KimTech sheet onto the sample of Supplale^{®} with a force of 2N, moving the sample at a speed of 10 cm/minute.

The sample of absorbent paper is then placed on the black part of a non-varnished BYK PA-2831 contrast sheet and the whole is photographed under identical lighting and distance conditions *(Canon PowerShot SX700 HS - 1120sec, ISO-200, F*/*4.5*).

The transfer is estimated by analysis of the levels of grey.

### Test results

The aerosols obtained from compositions 1 to 3 were sprayed under the conditions described above and the results obtained are described in table 3 below:

**Table 3**

| | **Composition 1 (according to the invention)** | **Composition 2 (according to the invention)** | **Composition 3 (outside the invention)** |
|---|---|---|---|
| Dry effect | +++ | +++ | + |
| Greasy effect | +++ | +++ | + |

| | | | |
|---|---|---|---|
| +++ dry: very good dry effect +++ greasy: no greasiness | | | |

The aerosols obtained from compositions 1 and 2 according to the invention make it possible to obtain a dry and non-greasy deposit, while at the same time conserving good deodorant efficacy.

The deposits obtained from compositions 1 and 2 according to the invention are drier and less greasy than that obtained from comparative composition 3.

## Claims

1. Anhydrous aerosol composition, having a water content of less than 5% by weight relative to the total weight of the composition, comprising:
- at least 0.3% by weight of active material of at least one deodorant active agent, relative to the total weight of the composition, the at least one deodorant active agent(s) comprising magnesium salts;
- at least two hydrocarbon-based oils, the at least two hydrocarbon-based oils comprising at least one hydrocarbon-based plant oil, and at least one fatty acid ester different from hydrocarbon-based plant oils; and
- at least one propellant;
**characterized in that** the composition comprises less than 0.5% by weight of silicone oil(s) and less than 0.1% by weight of modified clay(s), relative to the total weight of the composition, and **in that** the mass ratio of hydrocarbon-based oils relative to the total amount of oils present in the composition is between 0.85 and 1.00.

2. Composition according to Claim 1, **characterized in that** it comprises less than 0.2% by weight of silicone oil(s) relative to the total weight of the composition, and is preferably free of silicone oil(s).

3. Composition according to either of the preceding claims, **characterized in that** it is free of modified clay(s).

4. Composition according to any one of the preceding claims, **characterized in that** the mass ratio of hydrocarbon-based oils relative to the total amount of oils present in the composition is between 0.90 and 1.00 and is preferably equal to 1.

5. Composition according to any one of the preceding claims, **characterized in that** the deodorant active agent(s) are present in a content ranging from 0.3% to 10% by weight and preferably from 0.5% to 8% by weight of active material relative to the total weight of the composition; and/or
the hydrocarbon-based oils are present in a content ranging from 0.5% to 30% by weight, preferably from 1% to 20% by weight and even more preferentially from 1% to 18% by weight, relative to the total weight of the composition; and/or
the propellant(s) are present in a content ranging from 5% to 95% by weight, preferably from 20% to 95% by weight and better still from 40% to 95% by weight, relative to the total weight of the composition.

6. Composition according to any one of the preceding claims, **characterized in that** the deodorant active agent(s) are chosen from magnesium oxide, magnesium carbonate, magnesium hydroxide and magnesium bicarbonate, and mixtures thereof; said magnesium salt being more particularly magnesium oxide.

7. Composition according to any one of the preceding claims, **characterized in that** the content of hydrocarbon-based plant oil(s) is from 0.5% to 20% by weight, in particular from 1% to 15% by weight and more particularly from 1% to 10% by weight, relative to the total weight of the composition.

8. Composition according to any one of the preceding claims, **characterized in that** the at least one fatty acid ester is chosen from isopropyl palmitate, isopropyl myristate, isononyl isononanoate and C₁₂-C₁₅ alkyl benzoate, and mixtures thereof, and preferably from isopropyl palmitate and isopropyl myristate, and mixtures thereof, and more particularly isopropyl palmitate.

9. Composition according to any one of the preceding claims, **characterized in that** the at least one hydrocarbon-based plant oil is coconut oil.

10. Composition according to any one of the preceding claims, **characterized in that** it comprises from 0.5% to 20% by weight of fatty acid ester(s), preferably from 1% to 18% by weight of fatty acid ester(s) and better still from 2% to 15% by weight of fatty acid ester(s), relative to the total weight of the composition.

11. Composition according to any one of the preceding claims, **characterized in that** the mass ratio of fatty acid ester(s) relative to the total amount of oils present in the composition is between 0.55 and 0.85, in particular between 0.55 and 0.75 and more preferentially between 0.60 and 0.70.

12. Composition according to any one of the preceding claims, **characterized in that** it comprises less than 2% by weight of aluminium salts or complexes, more preferentially less than 1% by weight of aluminium salts or complexes, and better still less than 0.5% by weight of aluminium salts or complexes, relative to the total weight of the composition, and even more preferentially is free of aluminium salts or complexes.

13. Composition according to any one of the preceding claims, **characterized in that** the propellant is isobutane.

14. Composition according to any one of the preceding claims, **characterized in that** it comprises:
- at least 0.3% by weight of active material of deodorant active agent(s), the deodorant active agent(s) comprising magnesium salts, in particular magnesium oxide, relative to the total weight of the composition;
- at least one hydrocarbon-based plant oil, in particular coconut oil;
- at least one fatty acid ester, in particular isopropyl palmitate; and
- at least one propellant;
**characterized in that** the composition comprises less than 0.5% by weight of silicone oil(s) and less than 0.1% by weight of modified clay(s), relative to the total weight of the composition, and **in that** the mass ratio of fatty acid ester(s) relative to the total amount of oils present in the composition is between 0.55 and 0.85.

15. Process for the cosmetic treatment of body odour associated with human perspiration, notably underarm odour, and optionally human perspiration, comprising the step of applying a composition according to any one of the preceding claims to a skin surface.

## Patentansprüche

1. Wasserfreie Aerosolzusammensetzung mit einem Wassergehalt von weniger als 5 Gew.-%, bezogen auf das Gesamtgewicht der Zusammensetzung, umfassend:
- mindestens 0,3 Gew.-% des Wirkstoffs mindestens eines Deodorant-Wirkstoffs, bezogen auf das Gesamtgewicht der Zusammensetzung, wobei der mindestens eine Deodorant-Wirkstoff Magnesiumsalze umfasst,
- mindestens zwei Öle auf Kohlenwasserstoffbasis, wobei die mindestens zwei Öle auf Kohlenwasserstoffbasis mindestens ein Pflanzenöl auf Kohlenwasserstoffbasis und mindestens einen von Pflanzenölen auf Kohlenwasserstoffbasis verschiedenen Fettsäureester umfassen, und
- mindestens ein Treibmittel,
**dadurch gekennzeichnet, dass** die Zusammensetzung, bezogen auf das Gesamtgewicht der Zusammensetzung, weniger als 0,5 Gew.-% Silikonöl(e) und weniger als 0,1 Gew.-% modifizierten Ton/modifizierte Tone umfasst und dass das Massenverhältnis von Ölen auf Kohlenwasserstoffbasis zur Gesamtmenge an in der Zusammensetzung vorhandenen Ölen zwischen 0,85 und 1,00 liegt.

2. Zusammensetzung nach Anspruch 1, **dadurch gekennzeichnet, dass** sie, bezogen auf das Gesamtgewicht der Zusammensetzung, weniger als 0,2 Gew.-% Silikonöl(e) umfasst und vorzugsweise frei von Silikonöl(en) ist.

3. Zusammensetzung nach einem der vorhergehenden Ansprüche, **dadurch gekennzeichnet, dass** sie frei von modifiziertem Ton/modifizierten Tonen ist.

4. Zusammensetzung nach einem der vorhergehenden Ansprüche, **dadurch gekennzeichnet, dass** das Massenverhältnis von Ölen auf Kohlenwasserstoffbasis, bezogen auf die Gesamtmenge an in der Zusammensetzung enthaltenen Ölen, zwischen 0,90 und 1,00 liegt und vorzugsweise gleich 1 ist.

5. Zusammensetzung nach einem der vorhergehenden Ansprüche, **dadurch gekennzeichnet, dass** der Deodorant-Wirkstoff bzw. die Deodorant-Wirkstoffe in einem Gehalt im Bereich von 0,3 bis 10 Gew.-% und vorzugsweise 0,5 bis 8 Gew.-% an Wirkstoffmaterial, bezogen auf das Gesamtgewicht der Zusammensetzung, vorliegt bzw. vorliegen und/oder
die Öle auf Kohlenwasserstoffbasis in einem Gehalt im Bereich von 0,5 bis 30 Gew.-%, vorzugsweise 1 bis 20 Gew.-% und noch mehr bevorzugt 1 bis 18 Gew.-%, bezogen auf das Gesamtgewicht der Zusammensetzung, vorliegen und/oder
das/die Treibmittel in einem Gehalt im Bereich von 5 bis 95 Gew.-%, vorzugsweise 20 bis 95 Gew.-% und noch besser 40 bis 95 Gew.-%, bezogen auf das Gesamtgewicht der Zusammensetzung, vorliegt bzw. vorliegen.

6. Zusammensetzung nach einem der vorhergehenden Ansprüche, **dadurch gekennzeichnet, dass** der oder die Deodorant-Wirkstoffe aus Magnesiumoxid, Magnesiumcarbonat, Magnesiumhydroxid und Magnesiumhydrogencarbonat sowie Mischungen davon ausgewählt ist/sind, wobei das Magnesiumsalz insbesondere Magnesiumoxid ist.

7. Zusammensetzung nach einem der vorhergehenden Ansprüche, **dadurch gekennzeichnet, dass** der Gehalt des Pflanzenöls auf Kohlenwasserstoffbasis bzw. der Pflanzenöle auf Kohlenwasserstoffbasis im Bereich von 0,5 bis 20 Gew.-%, vorzugsweise 1 bis 15 Gew.-% und insbesondere 1 bis 10 Gew.-%, bezogen auf das Gesamtgewicht der Zusammensetzung, liegt.

8. Zusammensetzung nach einem der vorhergehenden Ansprüche, **dadurch gekennzeichnet, dass** der mindestens eine Fettsäureester aus Isopropylpalmitat, Isopropylmyristat, Isononylisononanoat und C₁₂-C₁₅-Alkylbenzoat und Mischungen davon, vorzugsweise aus Isopropylpalmitat und Isopropylmyristat und Mischungen davon und insbesondere Isopropylpalmitat ausgewählt ist.

9. Zusammensetzung nach einem der vorhergehenden Ansprüche, **dadurch gekennzeichnet, dass** es sich bei dem mindestens einen Pflanzenöl auf Kohlenwasserstoffbasis um Kokosnussöl handelt.

10. Zusammensetzung nach einem der vorhergehenden Ansprüche, **dadurch gekennzeichnet, dass** sie 0,5 bis 20 Gew.-% Fettsäureester, vorzugsweise 1 bis 18 Gew.-% Fettsäureester und noch besser 2 bis 15 Gew.-% Fettsäureester, bezogen auf das Gesamtgewicht der Zusammensetzung, umfasst.

11. Zusammensetzung nach einem der vorhergehenden Ansprüche, **dadurch gekennzeichnet, dass** das Massenverhältnis von Fettsäureester(n) zu der Gesamtmenge an in der Zusammensetzung enthaltenen Ölen zwischen 0,55 und 0,85, insbesondere zwischen 0,55 und 0,75 und besonders bevorzugt zwischen 0,60 und 0,70 liegt.

12. Zusammensetzung nach einem der vorhergehenden Ansprüche, **dadurch gekennzeichnet, dass** sie weniger als 2 Gew.-% Aluminiumsalze oder -komplexe, weiter bevorzugt weniger als 1 Gew.-% Aluminiumsalze oder -komplexe und noch besser weniger als 0,5 Gew.-% Aluminiumsalze oder - komplexe, bezogen auf das Gesamtgewicht der Zusammensetzung, umfasst und noch weiter bevorzugt frei von Aluminiumsalzen oder -komplexen ist.

13. Zusammensetzung nach einem der vorhergehenden Ansprüche, **dadurch gekennzeichnet, dass** es sich bei dem Treibmittel um Isobutan handelt.

14. Zusammensetzung nach einem der vorstehenden Ansprüche, **dadurch gekennzeichnet, dass** sie Folgendes umfasst:
- mindestens 0,3 Gew.-% des Wirkstoffs mindestens eines oder mehrerer Deodorant-Wirkstoffe, bezogen auf das Gesamtgewicht der Zusammensetzung, wobei der/die Deodorant-Wirkstoff(e) Magnesiumsalze, insbesondere Magnesiumoxid, umfasst/umfassen,
- mindestens ein Pflanzenöl auf Kohlenwasserstoffbasis, insbesondere Kokosnussöl,
- mindestens einen Fettsäureester, insbesondere Isopropylpalmitat, und
- mindestens ein Treibmittel,
**dadurch gekennzeichnet, dass** die Zusammensetzung, bezogen auf das Gesamtgewicht der Zusammensetzung, weniger als 0,5 Gew.-% Silikonöl(e) und weniger als 0,1 Gew.-% modifizierten Ton/modifizierte Tone umfasst und dass das Massenverhältnis von Fettsäureester(n) zur Gesamtmenge an in der Zusammensetzung vorhandenen Ölen zwischen 0,55 und 0,85 liegt.

15. Verfahren zur kosmetischen Behandlung von mit menschlicher Perspiration assoziiertem Körpergeruch, insbesondere Achselgeruch, und gegebenenfalls menschlicher Perspiration, umfassend den Schritt des Aufbringens einer Zusammensetzung nach einem der vorangehenden Ansprüche auf eine Hautoberfläche.

## Revendications

1. Composition d'aérosol anhydre, ayant une teneur en eau de moins de 5 % en poids, rapporté au poids total de la composition, comprenant :
- au moins 0,3 % en poids de matière active composée d'au moins un actif déodorant, rapporté au poids total de la composition, l'au moins un actif déodorant comprenant des sels de magnésium ;
- au moins deux huiles hydrocarbonées, les au moins deux huiles hydrocarbonées comprenant au moins une huile hydrocarbonée végétale, et au moins un ester d'acide gras différent des huiles hydrocarbonées végétales ; et
- au moins un propulseur ;
**caractérisée en ce que** la composition comprend moins de 0,5 % en poids d'huile(s) de silicone et moins de 0,1 % en poids d'argile(s) modifiée(s), rapporté au poids total de la composition, et **en ce que** le rapport massique entre les huiles hydrocarbonées et la quantité totale des huiles présentes dans la composition est compris entre 0,85 et 1,00.

2. Composition selon la revendication 1, **caractérisée en ce qu'**elle comprend moins de 0,2 % en poids d'huile(s) de silicone, rapporté au poids total de la composition, et est de préférence dépourvue d'huile(s) de silicone.

3. Composition selon l'une ou l'autre des revendications précédentes, **caractérisée en ce qu'**elle est dépourvue d'argile(s) modifiée(s).

4. Composition selon l'une quelconque des revendications précédentes, **caractérisée en ce que** le rapport massique entre les huiles hydrocarbonées et la quantité totale des huiles présentes dans la composition est compris entre 0,90 et 1,00 et de préférence est égal à 1.

5. Composition selon l'une quelconque des revendications précédentes, **caractérisée en ce que** le(s) actif(s) déodorant(s) est/sont présent(s) à une teneur allant de 0,3 % à 10 % en poids et de préférence de 0,5 % à 8 % en poids de matière active, rapporté au poids total de la composition ; et/ou
les huiles hydrocarbonées sont présentes à une teneur allant de 0,5 % à 30 % en poids, de préférence de 1 % à 20 % en poids, et mieux encore de 1 % à 18 % en poids, rapporté au poids total de la composition ; et/ou le(s) propulseur(s) est/sont présent(s) à une teneur allant de 5 % à 95 % en poids, de préférence de 20 % à 95 % en poids, et mieux encore de 40 % à 95 % en poids, rapporté au poids total de la composition.

6. Composition selon l'une quelconque des revendications précédentes, **caractérisée en ce que** le(s) actif(s) déodorant(s) est/sont choisi(s) parmi l'oxyde de magnésium, le carbonate de magnésium, l'hydroxyde de magnésium et le bicarbonate de magnésium, et les mélanges de ceux-ci ; ledit sel de magnésium étant plus particulièrement l'oxyde de magnésium.

7. Composition selon l'une quelconque des revendications précédentes, **caractérisée en ce que** la teneur en huile(s) hydrocarbonée(s) végétale(s) est de 0,5 % à 20 % en poids, en particulier de 1 % à 15 % en poids et plus particulièrement de 1 % à 10 % en poids, rapporté au poids total de la composition.

8. Composition selon l'une quelconque des revendications précédentes, **caractérisée en ce que** l'au moins un ester d'acide gras est choisi parmi le palmitate d'isopropyle, le myristate d'isopropyle, l'isononanoate d'isononyle, le benzoate d'alkyle en C₁₂-C₁₅, et les mélanges de ceux-ci, et de préférence parmi le palmitate d'isopropyle et le myristate d'isopropyle, et les mélanges de ceux-ci, et plus particulièrement le palmitate d'isopropyle.

9. Composition selon l'une quelconque des revendications précédentes, **caractérisée en ce que** l'au moins une huile hydrocarbonée végétale est l'huile de noix de coco.

10. Composition selon l'une quelconque des revendications précédentes, **caractérisée en ce qu'**elle comprend de 0,5 % à 20 % en poids d'ester(s) d'acide gras, de préférence de 1 % à 18 % en poids d'ester(s) d'acide gras et mieux encore de 2 % à 15 % en poids d'ester(s) d'acide gras, rapporté au poids total de la composition.

11. Composition selon l'une quelconque des revendications précédentes, **caractérisée en ce que** le rapport massique entre le(s) ester(s) d'acide gras et la quantité totale des huiles présentes dans la composition est compris entre 0,55 et 0,85, en particulier entre 0,55 et 0,75 et mieux encore entre 0,60 et 0,70.

12. Composition selon l'une quelconque des revendications précédentes, **caractérisée en ce qu'**elle comprend moins de 2 % en poids de sels ou complexes d'aluminium, mieux moins de 1 % en poids de sels ou complexes d'aluminium, et mieux encore moins de 0,5 % en poids de sels ou complexes d'aluminium, rapporté au poids total de la composition, et encore mieux est dépourvue de sels ou complexes d'aluminium.

13. Composition selon l'une quelconque des revendications précédentes, **caractérisée en ce que** le propulseur est l'isobutane.

14. Composition selon l'une quelconque des revendications précédentes, **caractérisée en ce qu'**elle comprend :
- au moins 0,3 % en poids de matière active composée d'actif(s) déodorant(s), le(s) actif(s) déodorant(s) comprenant des sels de magnésium, en particulier de l'oxyde de magnésium, rapporté au poids total de la composition ;
- au moins une huile hydrocarbonée végétale, en particulier de l'huile de coco ;
- au moins un ester d'acide gras, en particulier du palmitate d'isopropyle ; et
- au moins un propulseur ;
**caractérisée en ce que** la composition comprend moins de 0,5 % en poids d'huile(s) de silicone et moins de 0,1 % en poids d'argile(s) modifiée(s), rapporté au poids total de la composition, et **en ce que** le rapport massique entre le(s) ester(s) d'acide gras et la quantité totale des huiles présentes dans la composition est compris entre 0,55 et 0,85.

15. Procédé de traitement cosmétique de l'odeur corporelle associée à la transpiration humaine, notamment l'odeur des aisselles, et éventuellement la transpiration humaine, comprenant l'étape d'application d'une composition selon l'une quelconque des revendications précédentes sur une surface de peau.
